# EUROPEAN PATENT APPLICATION

(11) **EP 3 578 651 A2**
(43) Date of publication of application: **11.12.2019**
(21) Application number: 17882917.2
(22) Date of filing: 26.12.2017
(51) Int. Cl.: C12N 15/10, C12N 15/70, C12N 9/88, C12N 9/90, C12Q 1/04, C40B 30/06

(54) **RECOMBINANT STRAIN HAVING MODIFIED SUGAR METABOLIC PATHWAY AND METHOD FOR SCREENING SUGAR ISOMERASE USING SAME**

(30) Priority: 23.12.2016 KR 20160178419; 06.04.2017 KR 20170044740
(71) Applicant: Kyungpook National University Industry-Academic Cooperation Foundation, Daegu 41566 (KR)
(72) Inventor: LEE, Dong Woo, Daegu 41573 (KR); SHIN, Sun Mi, Daejeon 34952 (KR); JOO, Yun Hye, Yangsan-si Gyeongsangnam-do 50605 (KR); SUNG, Jae Yoon, Daegu 42221 (KR)
(74) Representative: J A Kemp LLP
(86) International application number: PCT/KR2017/015435
(87) International publication number: WO 2018/117773

(57) **Abstract**

The present invention relates to a recombinant strain having a modified sugar metabolic pathway resulting from the introduction of an enzyme derived from a different strain to a strain, and a high-speed screening method for various mutants and variants by which useful materials can be obtained or which can produce useful materials. The use of a recombinant vector and a strain according to the present invention not only can construct a new metabolic pathway in a strain to effectively obtain D-tagatose from D-galactose, but also can introduce randomly modified sugar isomerases and then allow D-galactose isomerase variants to be rapidly screened by conducting a cell growth-associated screening system.

## Description

### [Technical Field]

The present invention relates to a recombinant strain having a modified sugar metabolic pathway resulting from the introduction of an enzyme derived from a different strain to a strain, and a high-speed screening method for various mutants and variants which can use the same to obtain useful materials or which can produce useful materials.

### [Background Art]

L-arabinose isomerase (AI) (EC 5.3.1.5) is an enzyme that converts L-arabinose to L-ribulose *in vivo,* but converts D-galactose, structurally similar substrate to L-arabinose, into D-tagatose *in vitro.* Therefore, D-tagatose can be produced efficiently by using L-arabinose isomerase with high affinity and high activity for D-galactose.

D-tagatose is an isomer of D-galactose and is a natural saccharide present in fruits, milk, cheese and the like. D-tagatose has various health functional properties and sweetness very similar to sugar so that, when applied to various products, D-tagatose is used as a substitute sweetener that is healthy and tasty at the same time.

Meanwhile, directed evolution techniques have been used as an improved technique for converting the characteristics of enzymes to satisfy desired purposes such as enhancing the activity and structural stability of the enzyme or imparting an activity on a new substrate. One of the most common methods for producing mutant libraries to perform these techniques is to introduce random mutations by controlling the error rate of DNA polymerase during PCR using error-prone PCR method. The mutants are expressed, and then the mutants with excellent activity are selected to obtain improved enzymes. Developing effective screening technology for the characteristics and purpose of desired enzymes can be a core technology of the directed evolution.

There have been attempts to construct various mutants such as random mutation methods and point mutation methods to construct an enzyme modification library. However, these methods have limitations in applying the directed evolution technology to screen for 10⁷ or more mutants due to the absence of an ultra-fast screening method of modified enzymes. In other words, it is necessary to study a novel enzyme screening method that can replace the conventional methods which can not screen at high speed in order to apply the protein engineering technique of the sugar converting enzymes.

### [Disclosure]

### [Technical Problem]

To obtain a new enzyme for rare sugar, the present inventors have focused on the construction of novel metabolic pathways and a high-speed screening method of sugar converting enzymes. In this regard, it was confirmed that a gene encoding D-tagatose-1,6-bisphosphate aldolase (TBA) and a gene encoding L-arabinose isomerase (AI) are introduced into a non-galactose-utilizing stain so that it is possible to obtain a strain having a new sugar metabolic pathway and quickly to screen novel sugar isomerase by constructing the library in which mutation is randomly introduced into genes encoding AI and by measuring the strain growth using the library, thereby completing the present invention.

Therefore, the present invention aims to provide a method for rapidly screening novel strains having the isomerization activity of interest and the sugar metabolic activity using newly-constructed sugar metabolic pathway, thereby screening the novel mutant sugar isomerase with high speed.

### [Technical Solution]

In order to achieve the objects, the present invention provides a method of rapidly screening a mutant with the first sugar metabolic activity and the second sugar isomerization activity, the method including the steps of: 1) screening an enzyme essential for the first sugar metabolism through genomic analysis of the first sugar metabolic strain and a strain without the first sugar metabolic activity; 2) preparing a first sugar metabolic recombinant strain by introducing a gene encoding the enzyme screened in step 1) into a strain without the first sugar metabolic activity and the second sugar isomerization activity; 3) generating a second sugar isomerase mutant library by random mutagenesis; 4) introducing a gene mutant library obtained in step 3) into the first sugar metabolic recombinant strain prepared in step 2) to obtain a strain library; and 5) culturing a strain of the library obtained in step 4) in a defined medium supplemented with the second sugar as a sole carbon source and confirming the growth and growth rate of the strain, in which the first sugar and the second sugar are mutual isomers.

Further, the present invention provides a method of rapidly screening a mutant sugar isomerase with second sugar isomerization activity, the method including the steps of: 1) screening an enzyme essential for the first sugar metabolism through genomic analysis of the first sugar metabolic strain and a strain without the first sugar metabolic activity; 2) preparing a first sugar metabolic recombinant strain by introducing a gene encoding the enzyme screened in step 1) into a strain without the first sugar metabolic activity and the second sugar isomerization activity; 3) generating a second sugar isomerase mutant library by random mutagenesis; 4) introducing a gene mutant library obtained in step 3) into the first sugar metabolic recombinant strain prepared in step 2) to obtain a strain library; 5) culturing a strain of the library obtained in step 4) in a defined medium supplemented with the second sugar as a sole carbon source and screening the strain with faster growth rate; and 6) confirming a mutant enzyme introduced into the strain screened in step 5), in which the first sugar and the second sugar are mutual isomers.

Further, the present invention provides a recombinant vector containing a gene encoding D-tagatose-1,6-bisphosphate aldolase and a gene encoding L-arabinose isomerase.

Further, the present invention provides a recombinant strain with D-tagatose and D-galactose metabolic activity, in which the strain is introduced with a gene encoding D-tagatose-1,6-bisphosphate aldolase and a gene encoding L-arabinose isomerase.

Further, the present invention provides a method of preparing a recombinant strain with D-tagatose and D-galactose metabolic activity, the method including: introducing a gene encoding D-tagatose-1,6-bisphosphate aldolase and a gene encoding L-arabinose isomerase into a D-galactose non-metabolic strain.

Further, the present invention provides an L-arabinose isomerase variants with the sugar isomerization activity, the enzymes being represented by one amino acid sequence selected from the group consisting of SEQ ID NO. 2 to SEQ ID NO. 4.

### [Advantageous Effects]

The use of a high speed screening method, a recombinant vector and a strain according to the present invention not only can construct a new sugar metabolic pathway in a strain to effectively obtain D-tagatose from D-galactose, but also can introduce randomly modified sugar isomerases and then conduct a cell growth-associated screening system, thereby rapidly screening useful sugar isomerization mutant enzyme.

### [Description of Drawings]

FIG. 1 is a schematic diagram showing a novel sugar metabolic pathway introduced into a strain for constructing a cell growth-associated screening system according to the present invention.
FIG. 2 illustrates the growth curves of *Escherichia coli* cells in a complex medium (LB) and a defined medium (M9) in which glucose (glc) and D-galactose (gal) are added as a sole carbon source.
FIG. 3 illustrates the organization of the D-tagatose catabolic gene clusters of a microorganism with D-tagatose metabolic activity and of a microorganism without D-tagatose metabolic activity.
FIG. 4 is a schematic diagram of the expression vector of recombinant cloning of *gatY* gene related to D-tagatose metabolic activity.
FIG. 5 illustrates the results of SDS-PAGE analysis of protein expression by the foreign gene introduction by transfection of a *gatY* gene into *E. coli* (Lane 1: *E. coli* without *gatY* gene introduced and Lane 2: *E. coli* with *gatY* gene introduced).
FIG. 6 illustrates the growth curves of the strain to confirm D-tagatose metabolic activity in *E. coli* with *gatY* gene introduced.
FIG. 7 illustrates the colonies forming of *E. coli* with *gatY* gene in defined solid medium supplemented with tagatose as a sole carbon source to confirm the strain growth.
FIG. 8 is a schematic diagram of a pET-22b(+)-ECAI recombinant cloning expression vector.
FIG. 9 shows the results of SDS-PAGE analysis of the expression of *gatY* gene (31 kDa) and *araA* gene (56 kDa) encoding L-arabinose isomerase (AI).
FIG. 10 illustrates the growh curves of *E. coli* recombinant strain harboring a gene encoding D-tagatose-1,6-bisphosphate aldolase (TBA) and a gene encoding L-arabinose isomerase (AI) in medium supplemented with glucose or D-galactose and confirming the cell growth.
FIG. 11 illustrates a growth curves of *E. coli* harboring *araA* random mutagenesis library on D-galactose as carbon source for screening of improved encoding L-arabinose isomerase (AI) variants.
FIG. 12 illustrates the effects of mutations on L-arabinose isomerase (AI) activity selected by cell growth-associated high-speed screening system.

### [Modes of the Invention]

Unless otherwise defined herein, the terms have the meanings commonly used in the art to which the present invention pertains.

The present invention provides a method of rapidly screening a mutant with the first sugar metabolic activity and the second sugar isomerization activity, the method including the steps of: 1) screening an enzyme essential for the first sugar metabolism through genomic analysis of the first sugar metabolic strain and a strain without the first sugar metabolic activity; 2) preparing a first sugar metabolic recombinant strain by introducing a gene encoding the enzyme screened in step 1) into a strain without the first sugar metabolic activity and the second sugar isomerization activity; 3) generating a second sugar isomerase mutant library by random mutagenesis; 4) introducing a gene mutant library obtained in step 3) into the first sugar metabolic recombinant strain prepared in step 2) to obtain a strain library; and 5) culturing a strain of the library obtained in step 4) in a defined medium supplemented with the second sugar as a sole carbon source and confirming the growth and growth rate of the strain, in which the first sugar and the second sugar are mutual isomers.

The recombinant strain of the present invention is an expression host in which the sugar metabolic pathway is newly constructed to allow the second sugar isomerization activity and first sugar metabolic activity. The strain can isomerize the second sugar into first sugar by the newly constructed sugar metabolic pathway and can effectively metabolize the first sugar produced from the isomerization. Therefore, when the recombinant strain is cultured in a medium containing the second sugar as a sole carbon source, it can exhibit a high growth as compared with the wild type strain which does not change the sugar metabolism pathway. By the comparison of the growth rates, a strain containing an enzyme capable of effectively isomerizing the second sugar can be rapidly screened among the variants by random mutagenesis.

The first sugar and the second sugar in the isomeric relationship used in the present invention may include all conventional sugar isomers known in the art without limitation, but may preferably include a combination selected from the group consisting of a first combination in which the first sugar is tagatose and the second sugar is galactose; a second combination in which the first sugar is fructose and the second sugar is glucose; a third combination in which the first sugar is tagatose and the second sugar is fructose; a fourth combination in which the first sugar is mannose and the second sugar is glucose; a fifth combination in which the first sugar is glucose and the second sugar is galactose; and a sixth combination in which the first sugar is fructose and the second sugar is galactose.

One of ordinary skills in the art can select and use the first sugar and second sugar with the isomeric relationship suitable for constructing the sugar metabolic pathway necessary to obtain the desired mutant sugar isomerase. For example, an embodiment of the present invention discloses a method for obtaining a novel mutant isomerase capable of converting D-galactose (second sugar) into D-tagatose (first sugar). First, D-tagatose-1,6-bisphosphate aldolase (TBA), an enzyme capable of providing D-tagatose metabolic activity as the first sugar, is selected, and the gene *gatY* encoding the same is introduced into a strain without D-galactose metabolic activity. Then, in order to rapidly screen the mutant enzyme capable of isomerizing D-galactose, the second sugar, the L-arabinose isomerase (AI)-random mutant library is generated, and the gene library is introduced into the recombinant strain with tagatose metabolic activity with *gatY* gene introduced. When the recombinant strain is cultured in a medium containing D-galactose as the sole carbon source, it is possible to grow only the strain capable of effectively converting D-galactose to produce D-tagatose. Thus, the method of comparing strain growths may be used to rapidly screen a novel D-galactose mutant isomerase with D-galactose converting activity, in other words, a strain with L-arabinose (AI) mutant isomerase.

When conducting step 1) of screening an enzyme essential for the first sugar metabolism through genomic analysis of the first sugar metabolic strain and a strain without the first sugar metabolic activity, a person skilled in the art may use methods known in the art to carry out, without limitation, the comparison and genomic analysis of strains required to derive essential enzymes associated with metabolic activity of the desired first sugar.

When a gene encoding an enzyme essential for the first sugar metabolism such as D-tagatose-1,6-bisphosphate aldolase (TBA) is introduced as in the above steps 1) and 2), the sugar metabolic pathway is newly constructed in the strain without D-tagatose metabolic activity, which does not have strain growth in a conventional medium containing D-tagatose as a sole carbon source, and thus the strain growth can occur in the medium supplemented with D-tagatose.

Step 3) is a process of generating random mutations in the second sugar isomerase, for example, a gene encoding L-arabinose isomerase (AI) to obtain a gene mutant library. The term "random mutagenesis" means that any mutation is generated in a normal sequence as a template by an error-prone PCR, which is mutagenesis PCR. The term "gene mutant library" refers to a group or set of mutant genes obtained through random mutation.

Step 4) refers to a process of introducing a gene mutant library into the first sugar metabolic recombinant strain in step 2), for example, D-tagatose metabolic strain to obtain a strain library and means that D-tagatose metabolic strain additionally expresses mutant genes, thereby obtaining strain library in which the sugar metabolic pathway is newly constructed.

Each kind of individual mutant genes of the gene mutant library can be introduced into the D-tagatose metabolic strain of step 2). A strain library in which various mutant genes are newly introduced can be obtained through this process.

Step 5) is a process of culturing the strain of the strain library in a medium containing the second sugar as a sole carbon source to confirm the growth of the cell, which can observe the cell growth, thereby rapidly confirming whether the corresponding strain obtains the second sugar-converting ability. In particular, a strain showing excellent growth of the cell can be selected as a strain obtaining the second sugar-converting ability. The mutant gene of gene mutant library in step 3), which is introduced into the corresponding strain, is confirmed to derive a mutant with the second sugar isomerization activity imparting the second sugar-converting ability and the mutant enzyme introduced into the mutant.

Therefore, the present invention provides a method of rapidly screening a mutant sugar isomerase with the second sugar isomerization activity, the method including the steps of: 1) screening an enzyme essential for the first sugar metabolism through genomic analysis of the first sugar metabolic strain and a strain without the first sugar metabolic activity; 2) preparing a first sugar metabolic recombinant strain by introducing a gene encoding the enzyme screened in step 1) into a strain without the first sugar metabolic activity and the second sugar isomerization activity; 3) generating a second sugar isomerase mutant library by random mutagenesis; 4) introducing a gene mutant library obtained in step 3) into the first sugar metabolic recombinant strain prepared in step 2) to obtain a strain library; 5) culturing a strain of the library obtained in step 4) in a defined medium supplemented with the second sugar as a sole carbon source and screening the strain with faster growth rate; and 6) confirming a mutant enzyme introduced into the strain screened in step 5), in which the first sugar and the second sugar are mutual isomers.

Each step of the method can be performed according to the description of the method of rapidly screening the mutant with the second sugar isomerization activity and the first sugar metabolic activity as described above.

As disclosed in an embodiment of the present invention, the method of rapidly screening D-galactose isomerase mutants and L-arabinose isomerase mutants is characterized by being based on the cell growth-associated screening system.

The cell growth-associated screening system of the present invention means that a variety of candidate mutant genes are introduced into platform strains and that cell growth is confirmed, so that mutant genes showing activity based on this can be quickly selected and searched. More specifically, the present invention forms a L-arabinose isomerase mutant library, introduces them into a strain without D-galactose isomerization activity, strains are cultured in the defined medium containing D-galactose, and the growth of the strain is confirmed, thereby deriving particular mutant gene capable of effectively conferring D-galactose isomerization activity among a large number of mutant L-arabinose isomerase coding genes and using the same as D-galactose sugar isomerization mutant enzyme.

Further, the present invention provides a recombinant vector containing a gene encoding D-tagatose-1,6-bisphosphate aldolase and a gene encoding L-arabinose isomerase.

Further, the present invention provides a recombinant strain with D-galactose isomerization activity, in which the strain is introduced with a gene encoding D-tagatose-1,6-bisphosphate aldolase and a gene encoding L-arabinose isomerase.

The gene encoding the D-tagatose-1,6-bisphosphate aldolase may be a gene derived from a microorganism with tagatose metabolic activity, preferably a *gatY* gene (SEQ ID NO.: 5) derived from *Bacillus licheniformis* 14580. The amino acid sequence encoded thereby is represented by SEQ ID NO.: 7.

Further, the gene encoding L-arabinose isomerase of the present invention may be a gene derived from a microorganism with galactose metabolic activity, preferably an *araA* gene (SEQ ID NO.: 6) derived from *Escherichia coli.* The amino acid sequence encoded thereby is represented by SEQ ID NO.: 8.

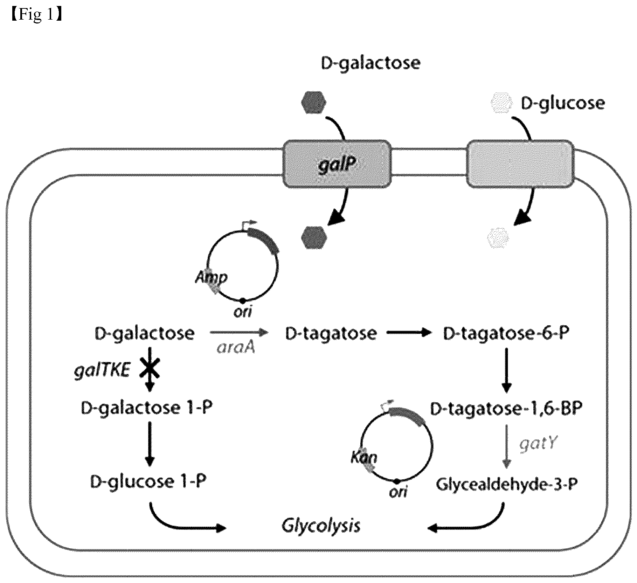

The recombinant vector containing the gene encoding D-tagatose-1,6-bisphosphate aldolase and the gene encoding L-arabinose isomerase of the present invention is used to obtain the recombinant strain with D-galactose metabolic activity into which the gene encoding D-tagatose-1,6-bisphosphate aldolase and the gene encoding L-arabinose isomerase are introduced. The foreign gene is introduced to construct a newly modified sugar metabolic pathway in the recombinant strain. Thus, the recombinant strain may have new metabolic activity which may produce useful D-tagatose using a sugar, preferably D-galactose, which has not been conventionally available. A novel sugar metabolic pathway constructed according to the introduction of such a gene is shown in FIG. 1 as a schematic diagram.

As used herein, the terms "sugar isomerization" and "sugar conversion" can be used interchangeably.

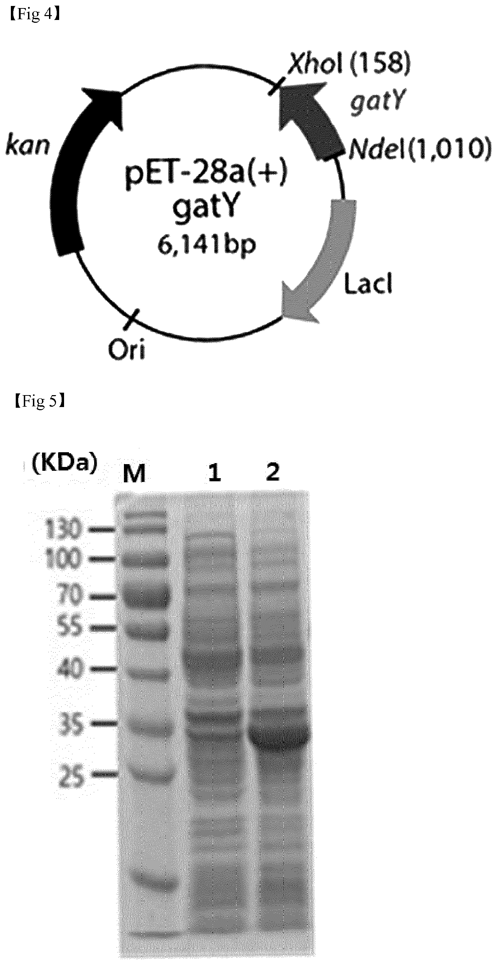

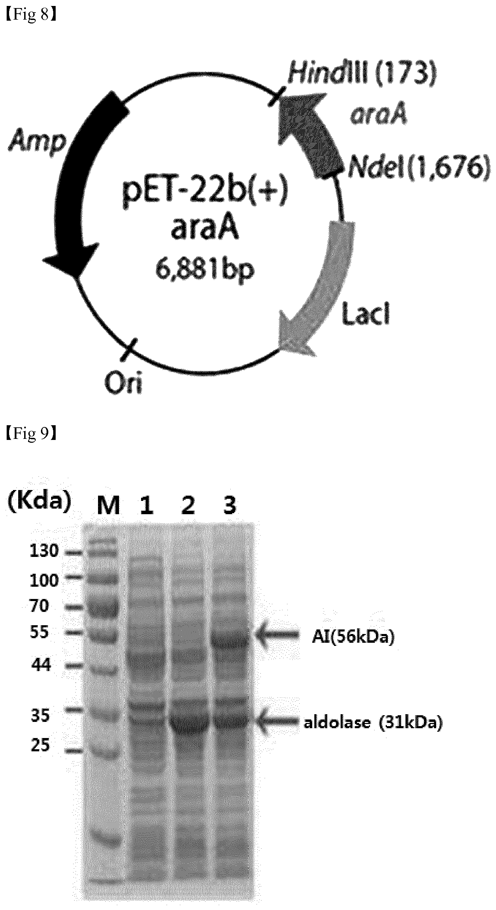

As used herein, the term "vector" refers to a carrier for introducing a foreign gene into a strain and may be preferably a plasmid. The plasmid may be any of those commonly used in the art. However, in particular, the plasmid having the sequence map shown in FIG. 4 may be used to introduce the *gatY* gene, and the plasmid having the sequence map shown in FIG. 8 may be used to introduce the *araA* gene.

The recombinant strain of the present invention is a strain newly having D-galactose metabolic activity by introducing a gene encoding D-tagatose-1,6-bisphosphate aldolase and a gene encoding L-arabinose isomerase, as a foreign gene, and in particular, the recombinant strain is a strain into which a gene is introduced without D-galactose metabolic activity. The strain without D-galactose metabolic activity is not particularly limited in its kind but may belong to one species selected from the group consisting of *Agrobacterium, Azospirillum Bacillus, Burkholderia, Clostridium, Enterobacter, Enterococcus, Escherichia, Flavobacterium, Klebsiella, Lactobacillus, Rhodobacter, Geobacillus,* and *Salmonella.* Particularly, *Escherichia coli* belonging to *Enterobacter,* which is most commonly used for recombination, is most preferable.

The recombinant strain with D-galactose metabolic activity, into which a gene encoding D-tagatose-1,6-bisphosphate aldolase and a gene encoding L-arabinose isomerase are introduced, is a strain capable of converting D-galactose into D-tagatose and using tagatose for glycolysis, which is characterized as a strain having both D-galactose and D-tagatose metabolic activity and assimilation ability.

Further, the present invention provides a method of preparing a recombinant strain with D-galactose isomerization activity, the method including: introducing a gene encoding D-tagatose-1,6-bisphosphate aldolase and a gene encoding L-arabinose isomerase into a D-galactose non-metabolic strain.

In the present invention, the method for introducing the two kinds of genes into the host is not particularly limited, but is preferably a process of inserting the genes into a vector and using recombinant DNA technology such as electrophoresis and heat shock transformation method to introduce genes into the recombinant microorganism, resulting in co-expression in the recombinant strain. Specific examples of vectors that can be used are shown in FIGS. 4 and 8.

When a gene encoding D-tagatose-1,6-bisphosphate aldolase and a gene encoding L-arabinose isomerase are introduced into a strain, the introduction of the two genes may be carried out simultaneously or sequentially. There are no restrictions on the order of sequential use.

Further, the present invention provides a method of producing D-tagatose, the method including: culturing the recombinant strain with D-galactose metabolic activity, into which the gene encoding D-tagatose-1,6-bisphosphate aldolase and the gene encoding L-arabinose isomerase are introduced, in a medium supplemented with D-galactose.

According to the method, although a convention strain without D-galactose metabolic activity is used, D-galactose can be metabolized through the process of introducing the gene encoding D-tagatose-1,6-bisphosphate aldolase and the gene encoding L-arabinose isomerase. Thus, D-tagatose, which a useful substance, may be produced in a medium containing D-galactose as a substrate.

The D-galactose-containing medium may be a medium containing components compatible with the growth of the recombinant strain and may be a defined medium containing only D-galactose and/or glucose as a sole carbon source.

Further, the present invention provides an L-arabinose isomerase mutant enzyme with sugar isomerization activity, the enzyme being represented by one amino acid sequence selected from the group consisting of SEQ ID NO. 2 to SEQ ID NO. 4. The mutant enzyme is a mutant amino sequence of the sugar isomerization enzyme mutant screened by the cell growth-associated screening system. The mutant enzyme is an enzyme derived by random mutagenesis, then introducing the mutant into the strain, then culturing the recombinant strain in medium containing D-galactose, and then confirming the growth.

The L-arabinose isomerase mutant enzyme with the sugar isomerization activity of the present invention may include, without limitation, a sequence having 90% to 99%, preferably 95% to 97% and more preferably 97% to 99% of homologous to an enzyme represented by each of SEQ ID NOS: 2, 3 and 4 as long as having equivalents of the functions of sugar isomerase.

Hereinafter, the present invention is described in detail with reference to examples. However, the following examples are only illustrative of the present invention and are not intended to limit the scope of the present invention.

### Example 1: Screening of platform host strain lacking the D-galactose metabolic pathway.

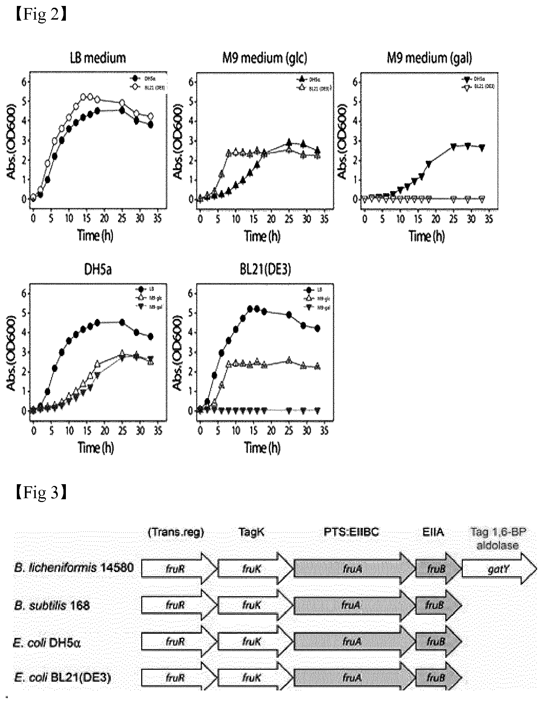

The host strain lacking the D-galactose catabolic pathway was screened and confirmed by the following experiments. The genomic analysis of *Escherichia coli* BL21 (DE3) lacking the D-galactose utilization pathway was carried out. The results indicated that the strain lacks three chromosomal genes, *galK* (EC 2.7.1.6 galactosekinase), *galT* (*EC* 2.7.1.12 galactose 1-phosphate uridylyltransferase), and *galE* (EC 5.1.3.2 UDP-galactose 4-epimerase), encoding the enzymes involved in D-galactose metabolic pathway of the carbohydrate metabolism. Based on this information, culture experiments were performed to experimentally confirm the cell growth on the following strains: *E. coli* BL21 (DE3) lacking the D-galactose metabolism-related genes and *E. coli* DH5α with the D-galactose metabolism-related genes. They were cultured in a complex medium (LB) and a defined medium (M9) containing either 5 g/L glucose (glc) or 5 g/L D-galactose (gal) for 35 hours. The results are shown in FIG. 2.

As shown in FIG. 2, *E. coli* DH5α was able to use glucose and D-galactose as a carbon source, while *E. coli* BL21 (DE3) did not show the cell growth in a D-galactose-containing medium.

### Example 2: Search for foreign genes essential for D-tagatose metabolism

In order to search for enzymes and genes essential for D-tagatose catabolic pathway, the direct comparison and analysis were conducted on the genomic information of the microorganism *Bacillus licheniformis* 14580 with D-tagatose metabolic activity and the microorganism *E. coli* BL21 (DE3) without D-tagatose metabolic activity. The results are shown in FIG. 3.

As shown in FIG. 3, it was confirmed that the microorganism *Bacillus licheniformis* 14580 with D-tagatose metabolic activity had *gatY* genes encoding D-tagatose 1, 6-bisphosphate aldolase in comparison with *E. coli* BL21 (DE3) without D-tagatose metabolic activity. Thus, they were selected as an essential enzyme for D-tagatose metabolism.

### Example 3: Construction of a recombinant E. coli strain growing on D-tagatose

### 3.1 Construction of GatY protein expression vector and E. coli transformants

An expression vector was constructed using the *gatY* gene expected to be essential for D-tagatose metabolism through Example 2 as described above. For the construction of the expression vector, the forward primer (Bli03552 *gatY Nde*I F5-CATATGCTGACAAATACGAAAAAAATGC-3) and the reverse primer (Bli03552 *gatY Xho*I R 5- CTCGAGTTAGTATCTGTCATTGCTCATGC-3) containing the respective restriction enzyme sequences (*Nde*I or *Xho*I) were used for PCR in pairs in accordance with the manufacture of the expression vector. For the gene amplification, PCR was carried out using genomic DNA of *Bacillus licheniformis* cells as a template. In particular, the primeSTAR HS DNA polymerase by Takara corporation was used. 30 cycles having 98°C for 30 secs, 57°C for 5 secs and 72°C for 1 min were carried out, followed by 72°C for 10 mins. The amplified PCR product was electrophoresed and then separated and purified using a gel extract kit (Qiagen). After cloning using the pTOP blunt V2 cloning kit (Enzynomics), DNA sequencing (Solgent) was performed to confirm whether the respective gene mutation was introduced. After confirming the absence of mutation in genes obtained by PCR, in order to prepare gene expression vectors, the pET-28(+) (Novagen) vector was treated with restriction enzymes (*Nde*I*, Xho*I)*,* and in order to recover genes cloned into the pTOP Blunt V2 vector, the vector was treated with restriction enzymes corresponding to the expression vector. They were purified and separated using electrophoresis and gel extraction kit (Qiagen). The restriction enzyme-treated vector and the recovered genes were ligated using a DNA ligation kit (promega) to prepare an expression vector pET-28a(+)-BL_TBA. A gene map of the expression vector is shown in FIG. 4. Further, in order to prepare an *E. coli* transformant for gene expression, the expression vector shown in FIG. 4 was introduced into *E. coli* BL21 (DE3) in which D-galactose non-metabolic activity was confirmed. In order to selectively culture the transformant, the transformant was plated on an LB solid medium containing kanamycin, an antibiotic suitable for the antibiotic resistance gene contained in the above expression vector having a final concentration of 50 µg/ml and was cultured at 37°C to form a single colony.

### 3.2 Identification of GatY protein expression in Escherichia coli

The recombinant vector pET-28a(+)-BL_TBA obtained in 3.1 as described above was transformed into *E. coli* BL21 (DE3) strain. The cells were pre-cultured, and 1% of pre-cultured cells were added to LB supplemented with kanamycin (50 µg/ml) in a flask. The primary culture was performed. The expression of *gatY* gene was induced for 6 hours at 37°C using IPTG (Isopropyl-D-1-thiogalactopyranoside) at a final concentration of 1 mM when the absorbance (600 nm) was 0.4 - 0.6.

The culture solution corresponding to 1 of the absorbance (600 nm) of cells inducing *gatY* protein expression was transferred to a 1.5 ml E-tube. After centrifugation, only the cultured cells were recovered. The cells were suspended in 100 µl of 1 × SDS sample buffer. After treatment in boiling water for 10 minutes, 12% SDS-PAGE (sodium dodecyl sulfate-polyacrylamide gel electrophoresis) analysis was performed, and the results are shown in FIG. 5.

As shown in FIG. 5, it was confirmed that *gatY* protein (30 kDa) was expressed and that the *gatY* gene was introduced.

### 3.3 Characterization of the growth phenotypes of E. coli expressing TBA on D-tagatose

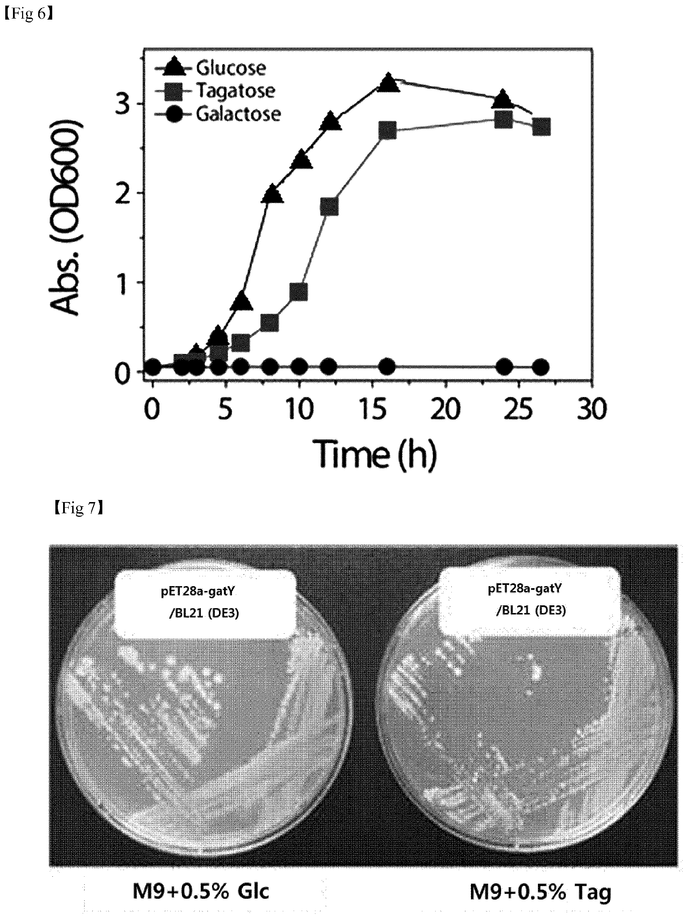

In order to confirm whether or not *E. coli* BL21 (DE3) host cells obtain D-tagatose metabolic activity according to the recombinant gene expression, the transformed new *E. coli* host cell pET-28a(+)-BL_TBA /*E. coli* BL21 (DE3) obtained in 3.1 and 3.2 as described above, were cultured in a defined (M9) medium containing 5 g/L D-tagatose as a sole carbon source, and the cell growth was monitored by measuring the absorbance at 600 nm. The results are shown in FIGS. 6 and 7.

As shown in FIG. 6, the transformant with the foreign gene, *gatY* introduced even in the D-tagatose-containing medium, showed excellent growth and non-proliferation rate similar to that of the glucose-containing medium. As shown in FIG. 7, it was confirmed that *E. coli* BL21 (DE3) host cells overexpressing the *gatY* foreign gene encoding TBA, showed the smooth cell growth according to the foreign protein expression. However, it did not show the cell growth in the defined medium containing 0.5% D-galactose as the sole carbon source. Therefore, it was confirmed that additional sugar metabolic pathway modification is necessary to impart D-galactose metabolic activity.

### Example 4: Preparation of an AI-activity-dependent, D-galactose utilizing strain

AI was expressed in the host cell prepared in Example 3, that is, lacking D-galactose metabolic activity and having metabolic activity for D-tagatose, in order to develop a platform strain and screening system for searching for a sugar isomerase library.

For this example, the protein expression vector for AI was prepared. For the construction of the expression vector, first, the forward primer (ECAI *Nde*I F, 5'-CATATGACGATTTTTGATAATTATGAAGTGTGG-3') and the reverse primer (ECAI *Hind*III R, 5'- AAGCTTTTAGCGACGAAACCCGTAATAC-3') containing the respective restriction enzyme sequences (*Nde*I or *Hind*III) were prepared and used for the gene amplification. For the gene amplification using PCR, genomic DNA of *E. coli* cells was used as a template. The primeSTAR HS DNA polymerase by Takara corporation was used. 30 cycles having 98°C for 30 secs, 57°C for 5 secs and 72°C for 1 min and 40 secs were carried out, followed by 72°C for 10 mins. The amplified PCR product was electrophoresed and then separated and purified using a gel extract kit (Qiagen). After cloning using the pTOP blunt V2 cloning kit (Enzynomics), DNA sequencing (Solgent) was performed to confirm whether the respective gene mutation was introduced. After confirming the absence of mutation in genes obtained by PCR, in order to prepare gene expression vectors, the pET-22b(+) (Novagen) vector was treated with restriction enzymes (*Nde*I*, Hind*III), and in order to recover genes cloned into the pTOP Blunt V2 vector, the vector was treated with restriction enzymes corresponding to the expression vector. They were purified and separated using electrophoresis and gel extraction kit (Qiagen). The restriction enzyme-treated vector and the recovered genes were ligated using a DNA ligation kit (promega) to prepare an expression vector pET-22b(+)-ECAI. A gene map of the expression vector prepared is shown in FIG. 8.

*E. coli* transformants were prepared as follows for the gene expression. The recombinant expression vector shown in FIG. 8 was introduced into *E. coli* pET-28a(+)-BL_TBA /BL21 (DE3) strain with D-tagatose metabolic activity, in which D-galactose non-metabolic activity was confirmed. In order to selectively culture the transformant, the transformant was plated on an LB solid medium containing ampicillin, an antibiotic suitable for the antibiotic resistance gene contained in the above expression vector having a final concentration of 100 µg/ml and was cultured at 37°C to form a single colony. After the cells were pre-cultured, 1% of pre-cultured cells were added to LB supplemented with ampicillin (100 µg/ml) in a flask. The primary culture was performed. The cells were mainly cultured. The expression of *araA* gene was induced for 6 hours at 37°C using IPTG (Isopropyl-D-1-thiogalactopyranoside) at a final concentration of 1 mM when the absorbance (600 nm) was 0.4 - 0.6. The culture solution corresponding to 1 of the absorbance (600 nm) of cells inducing protein expression was transferred to a 1.5 ml E-tube. After centrifugation, only the cultured cells were recovered. The cells were suspended in 100 µl of 1X SDS sample buffer. After treatment in boiling water for 10 minutes, 12% SDS-PAGE (sodium dodecyl sulfate-polyacrylamide gel electrophoresis) analysis was performed to confirm the expression of the *gatY* gene (31 kDa) encoding the D-tagatose 1, 6-bisphosphate aldolase enzyme and the expression of the *araA* gene (56 kDa) encoding the L-arabinose isomerase in *E. coli* as shown in FIG. 9.

As shown in FIG. 9, the *gatY* gene (31 kDa) encoding the D-tagatose 1, 6-bisphosphate aldolase enzyme and the *araA* gene (56 kDa) encoding the L-arabinose isomerase were expressed in the transformed *E. coli.*

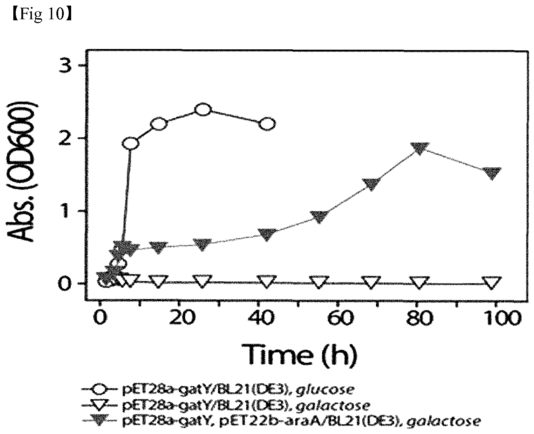

In order to assay the activity and cell growth of isomerase of the prepared cell growth-associated screening system, *E. coli* BL21 (DE3) in which pET-22b(+)-ECAI and pET-28(+)-*gatY* were transformed was cultured in a defined (M9) medium containing the antibiotics ampicillin (50 µg/ml) and kanamycin (25 µg/ml) suitable for the two plasmids, 0.05 g/L fructose, 0.45 g/L D-galactose and IPTG with a final concentration of 0.2 mM to confirm the cell growth curve of D-galactose isomerization activity of sugar isomerase. The results are shown in FIG. 10.

As shown in FIG. 10, it was confirmed that the strain in which the *gatY* gene encoding the D-tagatose 1, 6-bisphosphate aldolase enzyme and the *araA* gene encoding the L-arabinose isomerase were added in *E. coli* BL21 (DE3) strain which did not grow in the medium containing D-galactose showed the cell growth, and that D-galactose can be used as the sole carbon source due to the activity of the isomerase.

### Example 5: Screening of sugar isomerizing activity with the proto-type sugar isomerase and its variants

### 5.1 Generation of the araA mutants

In order to generate the *araA* gene mutants, mutagenic PCR was performed with a PCR random mutagenesis kit (Clontech, USA). 50 ng of the error-prone PCR library DNA was transformed into *E. coli* BL21 (DE3) containing plasmid pET-28a(+)-BL_TBA, and the cells were cultured in the defined (M9) medium containing 0.05 g/L fructose and 0.45% D-galactose. Thereafter, the formed colonies were collected, and the plasmid was extracted using a plasmid purification kit. 8 kinds of these plasmids were sequenced and their genetic diversity of the library was confirmed.

### 5.2 Screening of sugar isomerase variants using a cell growth-associated screening system

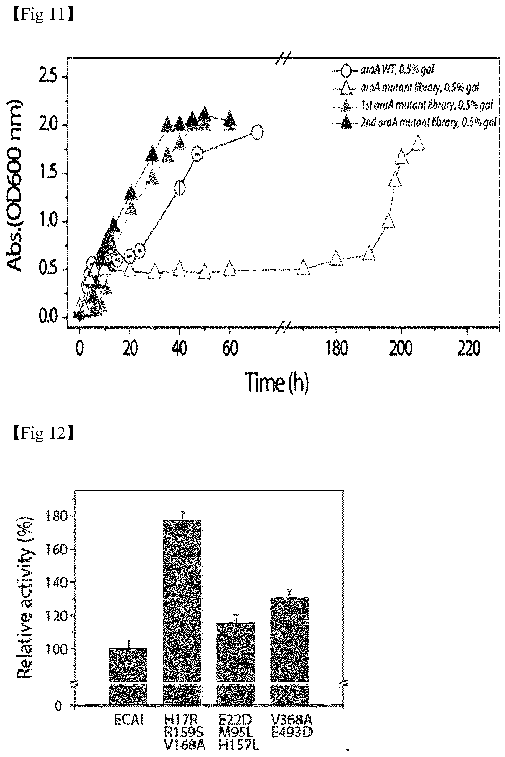

Recombinant *E. coli* BL21 (DE3) harboring pET-28a(+)-BL_TBA were transformed with *araA* genetic libraries(i.e., pET-22b(+)-ECAI_Var). Transformants harboring pET-28a(+)-BL_TBA and pET-22b(+)-ECAI_Var were grown in the defined medium (M9) containing 0.05 g/L fructose and 0.45 g/L D-galactose as a carbon source and IPTG with a final concentration of 0.2 mM, and the cell growth was confirmed. The results are shown in FIG. 11.

As shown in FIG. 11, it was confirmed that as the protein expression of the mutant gene library was varied, the level of the cell growth and the non-proliferation rate were different. These indicate that the ability to utilize D-galactose as a carbon source may vary depending on the introduction of the *araA* gene and its mutation.

### 5.3. Verification of D-galactose availability of sugar isomerase variants

The plasmids were isolated from the sugar isomerase variants and the wild type selected by the cell growth-associated screening system of 5.2 above as follows: pET-22b(+)-ECAI H17R/R159S/V168A, pET-22b(+)-ECAI E22D/M95L/H157L, and pET-22b(+)-ECAI-V368A/E493D. Further, the sequencing of the isolated plasmids was performed to analyze the mutant amino acid sequences of the mutants selected by the cell growth-associated screening system. They are represented by SEQ ID NOS.: 2 to 4, respectively. The amino acid sequence of the wild-type ECAI is represented by SEQ ID NO.: 1.

In addition, Ni2+ column chromatography was performed to purely isolate the mutant enzymes, and the activity of the mutant enzymes was compared with the ECAI wild type enzyme. That is, the activity of host strain, ECAI wild-type and sugar isomerization enzyme variants on D-galactose was measured and compared, and the results are shown in FIG. 12.

As shown in FIG. 12, it was confirmed that pET-22b(+)-ECAI H17R/R159S/V168A-derived mutant enzyme (SEQ ID NO.: 2), pET-22b(+)-ECAI E22D/M95L/H157L-derived mutant enzyme (SEQ ID NO.: 3), and pET-22b(+)-ECAI-V368A/E493D-derived mutant enzyme (SEQ ID NO.: 4) increased the activity to D-galactose up to 80%.

These results indicate that when a mutant library of L-arabinose isomerase (AI) by random mutagenesis was generated, then the library was introduced into a strain and form strain library. And then D-galactose metabolic activity was assayed through cell growth, specific mutagenic enzymes and sugar isomerase, L-arabinose isomerase variants that can impart D-galactose metabolic activity can be rapidly selected.

Although the present invention has been described in terms of the preferred embodiments as described above, it is possible to make various modifications and variations without departing from the spirit and scope of the present invention. It is also to be understood that the appended claims are intended to cover such modifications and variations falling within the scope of the present invention.

## Claims

1. A method of rapidly screening a mutant with first sugar metabolic activity and second sugar isomerization activity, the method comprising the steps of:
1) screening an enzyme essential for first sugar metabolism through genomic analysis of a first sugar metabolic strain and a strain without the first sugar metabolic activity;
2) preparing a first sugar metabolic recombinant strain by introducing a gene encoding the enzyme screened in step 1) into a strain without the first sugar metabolic activity and the second sugar isomerization activity;
3) generating a second sugar isomerase mutant library by random mutagenesis;
4) introducing a gene mutant library obtained in step 3) into the first sugar metabolic recombinant strain prepared in step 2) to obtain a strain library; and
5) culturing a strain of the library obtained in step 4) in a defined medium supplemented with the second sugar as a sole carbon source and confirming the growth and growth rate of the strain,
wherein the first sugar and the second sugar are mutual isomers.

2. The method of claim 1, wherein the first sugar and the second sugar are a combination selected from the group consisting of a first combination in which the first sugar is tagatose and the second sugar is galactose; a second combination in which the first sugar is fructose and the second sugar is glucose; a third combination in which the first sugar is tagatose and the second sugar is fructose; a fourth combination in which the first sugar is mannose and the second sugar is glucose; a fifth combination in which the first sugar is glucose and the second sugar is galactose; and a sixth combination in which the first sugar is fructose and the second sugar is galactose.

3. The method of claim 2, wherein the first sugar is tagatose and the second sugar is galactose,
wherein the enzyme screened in step 1) is D-tagatose-1,6-bisphosphate aldolase, and
wherein the isomerase in step 3) is L-arabinose isomerase.

4. The method of claim 1, wherein the random mutagenesis in step 3) is carried out by an error-prone PCR.

5. A method of rapidly screening a mutant sugar isomerase with a second sugar isomerization activity, the method comprising the steps of:
1) screening an enzyme essential for first sugar metabolism through genomic analysis of a first sugar metabolic strain and a strain without first sugar metabolic activity;
2) preparing a first sugar metabolic recombinant strain by introducing a gene encoding the enzyme screened in step 1) into a strain without the first sugar metabolic activity and the second sugar isomerization activity;
3) generating a second sugar isomerase mutant library by random mutagenesis;
4) introducing a gene mutant library obtained in step 3) into the first sugar metabolic recombinant strain prepared in step 2) to obtain a strain library;
5) culturing a strain of the library obtained in step 4) in a defined medium supplemented with the second sugar as a sole carbon source and screening the strain with faster growth rate; and
6) confirming a mutant enzyme introduced into the strain screened in step 5),
wherein the first sugar and the second sugar are mutual isomers.

6. The method of claim 5, wherein the first sugar and the second sugar are a combination selected from the group consisting of a first combination in which the first sugar is tagatose and the second sugar is galactose; a second combination in which the first sugar is fructose and the second sugar is glucose; a third combination in which the first sugar is tagatose and the second sugar is fructose; a fourth combination in which the first sugar is mannose and the second sugar is glucose; a fifth combination in which the first sugar is glucose and the second sugar is galactose; and a sixth combination in which the first sugar is fructose and the second sugar is galactose.

7. A recombinant vector containing a gene encoding D-tagatose-1,6-bisphosphate aldolase and a gene encoding L-arabinose isomerase.

8. The recombinant vector of claim 7, the gene encoding D-tagatose-1,6-bisphosphate aldolase is *gatY* gene.

9. The recombinant vector of claim 7, the gene encoding L-arabinose isomerase is *araA* gene.

10. A recombinant strain with D-galactose isomerization activity, wherein the strain is introduced with a gene encoding D-tagatose-1,6-bisphosphate aldolase and a gene encoding L-arabinose isomerase.

11. A method of preparing a recombinant strain with D-galactose isomerization activity, the method comprising:
introducing a gene encoding D-tagatose-1,6-bisphosphate aldolase and a gene encoding L-arabinose isomerase into a D-galactose non-metabolic strain.

12. A method of producing D-tagatose, the method comprising:
culturing the recombinant strain of claim 10 in a medium supplemented with D-galactose.

13. An L-arabinose isomerase variants with D-galactose isomerization activity, the enzyme being represented by one amino acid sequence selected from the group consisting of SEQ ID NO. 2 to SEQ ID NO. 4.


<110>    Kyungpook National University Industry-Academic Cooperation
Foundation

<120>    Screening methods and compositions for sugar isomerizing enzymes
         using engineered recombinant strains with modified sugar
         metabolic pathways

<130>    KNU1-47PCT

<150>    KR 10-2017-0044740
<151>    2017-04-06

<150>    KR 10-2016-0178419
<151>    2016-12-23

<160>    12

<170>    KoPatentIn 3.0

<210>    1
<211>    500
<212>    PRT
<213>    Artificial Sequence

<220>
<223>    ECAI


<400>    1
Met Thr Ile Phe Asp Asn Tyr Glu Val Trp Phe Val Ile Gly Ser Gln
  1               5                  10                  15

His Leu Tyr Gly Pro Glu Thr Leu Arg Gln Val Thr Gln His Ala Glu
             20                  25                  30

His Val Val Asn Ala Leu Asn Thr Glu Ala Lys Leu Pro Cys Lys Leu
         35                  40                  45

Val Leu Lys Pro Leu Gly Thr Thr Pro Asp Glu Ile Thr Ala Ile Cys
     50                  55                  60

Arg Asp Ala Asn Tyr Asp Asp Arg Cys Ala Gly Leu Val Val Trp Leu
 65                  70                  75                  80

His Thr Phe Ser Pro Ala Lys Met Trp Ile Asn Gly Leu Thr Met Leu
                 85                  90                  95

Asn Lys Pro Leu Leu Gln Phe His Thr Gln Phe Asn Ala Ala Leu Pro
            100                 105                 110

Trp Asp Ser Ile Asp Met Asp Phe Met Asn Leu Asn Gln Thr Ala His
        115                 120                 125

Gly Gly Arg Glu Phe Gly Phe Ile Gly Ala Arg Met Arg Gln Gln His
    130                 135                 140

Ala Val Val Thr Gly His Trp Gln Asp Lys Gln Ala His Glu Arg Ile
145                 150                 155                 160

Gly Ser Trp Met Arg Gln Ala Val Ser Lys Gln Asp Thr Arg His Leu
                165                 170                 175

Lys Val Cys Arg Phe Gly Asp Asn Met Arg Glu Val Ala Val Thr Asp

180                 185                 190

Gly Asp Lys Val Ala Ala Gln Ile Lys Phe Gly Phe Ser Val Asn Thr
    195                 200                 205

Trp Ala Val Gly Asp Leu Val Gln Val Val Asn Ser Ile Ser Asp Gly
    210                 215                 220

Asp Val Asn Ala Leu Val Asp Glu Tyr Glu Ser Cys Tyr Thr Met Thr
225                 230                 235                 240

Pro Ala Thr Gln Ile His Gly Lys Lys Arg Gln Asn Val Leu Glu Ala
                245                 250                 255

Ala Arg Ile Glu Leu Gly Met Lys Arg Phe Leu Glu Gln Gly Gly Phe
            260                 265                 270

His Ala Phe Thr Thr Thr Phe Glu Asp Leu His Gly Leu Lys Gln Leu
        275                 280                 285

Pro Gly Leu Ala Val Gln Arg Leu Met Gln Gln Gly Tyr Gly Phe Ala
    290                 295                 300

Gly Glu Gly Asp Trp Lys Thr Ala Ala Leu Leu Arg Ile Met Lys Val
305                 310                 315                 320

Met Ser Thr Gly Leu Gln Gly Gly Thr Ser Phe Met Glu Asp Tyr Thr
                325                 330                 335

Tyr His Phe Glu Lys Gly Asn Asp Leu Val Leu Gly Ser His Met Leu
            340                 345                 350

Glu Val Cys Pro Ser Ile Ala Ala Glu Glu Lys Pro Ile Leu Asp Val
        355                 360                 365

Gln His Leu Gly Ile Gly Gly Lys Asp Asp Pro Ala Arg Leu Ile Phe
    370                 375                 380

Asn Thr Gln Thr Gly Pro Ala Ile Val Ala Ser Leu Ile Asp Leu Gly
385                 390                 395                 400

Asp Arg Tyr Arg Leu Leu Val Asn Cys Ile Asp Thr Val Lys Thr Pro
                405                 410                 415

His Ser Leu Pro Lys Leu Pro Val Ala Asn Ala Leu Trp Lys Ala Gln
            420                 425                 430

Pro Asp Leu Pro Thr Ala Ser Glu Ala Trp Ile Leu Ala Gly Gly Ala
        435                 440                 445

His His Thr Val Phe Ser His Ala Leu Asn Leu Asn Asp Met Arg Gln
    450                 455                 460

Phe Ala Glu Met His Asp Ile Glu Ile Thr Val Ile Asp Asn Asp Thr
465                 470                 475                 480

Arg Leu Pro Ala Phe Lys Asp Ala Leu Arg Trp Asn Glu Val Tyr Tyr
                485                 490                 495

Gly Phe Arg Arg
            500

<210>    2
<211>    500
<212>    PRT
<213>    Artificial Sequence

<220>
<223>    ECAI H17R/R159S/V168A


<400>    2
Met Thr Ile Phe Asp Asn Tyr Glu Val Trp Phe Val Ile Gly Ser Gln
1               5                   10                  15

Arg Leu Tyr Gly Pro Glu Thr Leu Arg Gln Val Thr Gln His Ala Glu
            20                  25                  30

His Val Val Asn Ala Leu Asn Thr Glu Ala Lys Leu Pro Cys Lys Leu
        35                  40                  45

Val Leu Lys Pro Leu Gly Thr Thr Pro Asp Glu Ile Thr Ala Ile Cys
    50                  55                  60

Arg Asp Ala Asn Tyr Asp Asp Arg Cys Ala Gly Leu Val Val Trp Leu
65                  70                  75                  80

His Thr Phe Ser Pro Ala Lys Met Trp Ile Asn Gly Leu Thr Met Leu
                85                  90                  95

Asn Lys Pro Leu Leu Gln Phe His Thr Gln Phe Asn Ala Ala Leu Pro
            100                 105                 110

Trp Asp Ser Ile Asp Met Asp Phe Met Asn Leu Asn Gln Thr Ala His
        115                 120                 125

Gly Gly Arg Glu Phe Gly Phe Ile Gly Ala Arg Met Arg Gln Gln His
    130                 135                 140

Ala Val Val Thr Gly His Trp Gln Asp Lys Gln Ala His Glu Ser Ile
145                 150                 155                 160

Gly Ser Trp Met Arg Gln Ala Ala Ser Lys Gln Asp Thr Arg His Leu
                165                 170                 175

Lys Val Cys Arg Phe Gly Asp Asn Met Arg Glu Val Ala Val Thr Asp
            180                 185                 190

Gly Asp Lys Val Ala Ala Gln Ile Lys Phe Gly Phe Ser Val Asn Thr
        195                 200                 205

Trp Ala Val Gly Asp Leu Val Gln Val Val Asn Ser Ile Ser Asp Gly
    210                 215                 220

Asp Val Asn Ala Leu Val Asp Glu Tyr Glu Ser Cys Tyr Thr Met Thr
225                 230                 235                 240

Pro Ala Thr Gln Ile His Gly Lys Lys Arg Gln Asn Val Leu Glu Ala
                245                 250                 255

Ala Arg Ile Glu Leu Gly Met Lys Arg Phe Leu Glu Gln Gly Gly Phe
            260                 265                 270

His Ala Phe Thr Thr Thr Phe Glu Asp Leu His Gly Leu Lys Gln Leu
        275                 280                 285

Pro Gly Leu Ala Val Gln Arg Leu Met Gln Gln Gly Tyr Gly Phe Ala
    290                 295                 300

Gly Glu Gly Asp Trp Lys Thr Ala Ala Leu Leu Arg Ile Met Lys Val
305                 310                 315                 320

Met Ser Thr Gly Leu Gln Gly Gly Thr Ser Phe Met Glu Asp Tyr Thr
                325                 330                 335

Tyr His Phe Glu Lys Gly Asn Asp Leu Val Leu Gly Ser His Met Leu
            340                 345                 350

Glu Val Cys Pro Ser Ile Ala Ala Glu Glu Lys Pro Ile Leu Asp Val
        355                 360                 365

Gln His Leu Gly Ile Gly Gly Lys Asp Asp Pro Ala Arg Leu Ile Phe
    370                 375                 380

Asn Thr Gln Thr Gly Pro Ala Ile Val Ala Ser Leu Ile Asp Leu Gly
385                 390                 395                 400

Asp Arg Tyr Arg Leu Leu Val Asn Cys Ile Asp Thr Val Lys Thr Pro
                405                 410                 415

His Ser Leu Pro Lys Leu Pro Val Ala Asn Ala Leu Trp Lys Ala Gln
            420                 425                 430

Pro Asp Leu Pro Thr Ala Ser Glu Ala Trp Ile Leu Ala Gly Gly Ala
        435                 440                 445

His His Thr Val Phe Ser His Ala Leu Asn Leu Asn Asp Met Arg Gln
    450                 455                 460

Phe Ala Glu Met His Asp Ile Glu Ile Thr Val Ile Asp Asn Asp Thr
465                 470                 475                 480

Arg Leu Pro Ala Phe Lys Asp Ala Leu Arg Trp Asn Glu Val Tyr Tyr
                485                 490                 495

Gly Phe Arg Arg
            500


<210>    3
<211>    500
<212>    PRT
<213>    Artificial Sequence

<220>
<223>    ECAI E22D/M95L/H157L


<400>    3
Met Thr Ile Phe Asp Asn Tyr Glu Val Trp Phe Val Ile Gly Ser Gln
1               5                   10                  15

His Leu Tyr Gly Pro Asp Thr Leu Arg Gln Val Thr Gln His Ala Glu
            20                  25                  30

His Val Val Asn Ala Leu Asn Thr Glu Ala Lys Leu Pro Cys Lys Leu
        35                  40                  45

Val Leu Lys Pro Leu Gly Thr Thr Pro Asp Glu Ile Thr Ala Ile Cys
        50                  55                  60

Arg Asp Ala Asn Tyr Asp Asp Arg Cys Ala Gly Leu Val Val Trp Leu
65                  70                  75                  80

His Thr Phe Ser Pro Ala Lys Met Trp Ile Asn Gly Leu Thr Leu Leu
                85                  90                  95

Asn Lys Pro Leu Leu Gln Phe His Thr Gln Phe Asn Ala Ala Leu Pro
            100                 105                 110

Trp Asp Ser Ile Asp Met Asp Phe Met Asn Leu Asn Gln Thr Ala His
        115                 120                 125

Gly Gly Arg Glu Phe Gly Phe Ile Gly Ala Arg Met Arg Gln Gln His
    130                 135                 140

Ala Val Val Thr Gly His Trp Gln Asp Lys Gln Ala Leu Glu Arg Ile
145                 150                 155                 160

Gly Ser Trp Met Arg Gln Ala Val Ser Lys Gln Asp Thr Arg His Leu
                165                 170                 175

Lys Val Cys Arg Phe Gly Asp Asn Met Arg Glu Val Ala Val Thr Asp
            180                 185                 190

Gly Asp Lys Val Ala Ala Gln Ile Lys Phe Gly Phe Ser Val Asn Thr
        195                 200                 205

Trp Ala Val Gly Asp Leu Val Gln Val Val Asn Ser Ile Ser Asp Gly
    210                 215                 220

Asp Val Asn Ala Leu Val Asp Glu Tyr Glu Ser Cys Tyr Thr Met Thr
225                 230                 235                 240

Pro Ala Thr Gln Ile His Gly Lys Lys Arg Gln Asn Val Leu Glu Ala
                245                 250                 255

Ala Arg Ile Glu Leu Gly Met Lys Arg Phe Leu Glu Gln Gly Gly Phe
            260                 265                 270

His Ala Phe Thr Thr Thr Phe Glu Asp Leu His Gly Leu Lys Gln Leu
        275                 280                 285

Pro Gly Leu Ala Val Gln Arg Leu Met Gln Gln Gly Tyr Gly Phe Ala
    290                 295                 300

Gly Glu Gly Asp Trp Lys Thr Ala Ala Leu Leu Arg Ile Met Lys Val
305                 310                 315                 320

Met Ser Thr Gly Leu Gln Gly Gly Thr Ser Phe Met Glu Asp Tyr Thr
                325                 330                 335

Tyr His Phe Glu Lys Gly Asn Asp Leu Val Leu Gly Ser His Met Leu
            340                 345                 350

Glu Val Cys Pro Ser Ile Ala Ala Glu Glu Lys Pro Ile Leu Asp Val
        355                 360                 365

Gln His Leu Gly Ile Gly Gly Lys Asp Asp Pro Ala Arg Leu Ile Phe
    370                 375                 380

Asn Thr Gln Thr Gly Pro Ala Ile Val Ala Ser Leu Ile Asp Leu Gly
385                 390                 395                 400

Asp Arg Tyr Arg Leu Leu Val Asn Cys Ile Asp Thr Val Lys Thr Pro
                405                 410                 415

His Ser Leu Pro Lys Leu Pro Val Ala Asn Ala Leu Trp Lys Ala Gln
            420                 425                 430

Pro Asp Leu Pro Thr Ala Ser Glu Ala Trp Ile Leu Ala Gly Gly Ala
        435                 440                 445

His His Thr Val Phe Ser His Ala Leu Asn Leu Asn Asp Met Arg Gln
    450                 455                 460

Phe Ala Glu Met His Asp Ile Glu Ile Thr Val Ile Asp Asn Asp Thr
465                 470                 475                 480

Arg Leu Pro Ala Phe Lys Asp Ala Leu Arg Trp Asn Glu Val Tyr Tyr
                485                 490                 495

Gly Phe Arg Arg
            500


<210>    4
<211>    500
<212>    PRT
<213>    Artificial Sequence

<220>
<223>    ECAI V368A/E493D


<400>    4
Met Thr Ile Phe Asp Asn Tyr Glu Val Trp Phe Val Ile Gly Ser Gln
1               5                   10                  15

His Leu Tyr Gly Pro Glu Thr Leu Arg Gln Val Thr Gln His Ala Glu
            20                  25                  30

His Val Val Asn Ala Leu Asn Thr Glu Ala Lys Leu Pro Cys Lys Leu
        35                  40                  45

Val Leu Lys Pro Leu Gly Thr Thr Pro Asp Glu Ile Thr Ala Ile Cys
    50                  55                  60

Arg Asp Ala Asn Tyr Asp Asp Arg Cys Ala Gly Leu Val Val Trp Leu
65                  70                  75                  80

His Thr Phe Ser Pro Ala Lys Met Trp Ile Asn Gly Leu Thr Met Leu
                85                  90                  95

Asn Lys Pro Leu Leu Gln Phe His Thr Gln Phe Asn Ala Ala Leu Pro
            100                 105                 110

Trp Asp Ser Ile Asp Met Asp Phe Met Asn Leu Asn Gln Thr Ala His
        115                 120                 125

Gly Gly Arg Glu Phe Gly Phe Ile Gly Ala Arg Met Arg Gln Gln His
    130                 135                 140

Ala Val Val Thr Gly His Trp Gln Asp Lys Gln Ala His Glu Arg Ile

145                 150                 155                 160

Gly Ser Trp Met Arg Gln Ala Val Ser Lys Gln Asp Thr Arg His Leu
                165                 170                 175

Lys Val Cys Arg Phe Gly Asp Asn Met Arg Glu Val Ala Val Thr Asp
            180                 185                 190

Gly Asp Lys Val Ala Ala Gln Ile Lys Phe Gly Phe Ser Val Asn Thr
        195                 200                 205

Trp Ala Val Gly Asp Leu Val Gln Val Val Asn Ser Ile Ser Asp Gly
    210                 215                 220

Asp Val Asn Ala Leu Val Asp Glu Tyr Glu Ser Cys Tyr Thr Met Thr
225                 230                 235                 240

Pro Ala Thr Gln Ile His Gly Lys Lys Arg Gln Asn Val Leu Glu Ala
                245                 250                 255

Ala Arg Ile Glu Leu Gly Met Lys Arg Phe Leu Glu Gln Gly Gly Phe
            260                 265                 270

His Ala Phe Thr Thr Thr Phe Glu Asp Leu His Gly Leu Lys Gln Leu
        275                 280                 285

Pro Gly Leu Ala Val Gln Arg Leu Met Gln Gln Gly Tyr Gly Phe Ala
    290                 295                 300

Gly Glu Gly Asp Trp Lys Thr Ala Ala Leu Leu Arg Ile Met Lys Val
305                 310                 315                 320

Met Ser Thr Gly Leu Gln Gly Gly Thr Ser Phe Met Glu Asp Tyr Thr
                325                 330                 335

Tyr His Phe Glu Lys Gly Asn Asp Leu Val Leu Gly Ser His Met Leu
            340                 345                 350

Glu Val Cys Pro Ser Ile Ala Ala Glu Glu Lys Pro Ile Leu Asp Ala
        355                 360                 365

Gln His Leu Gly Ile Gly Gly Lys Asp Asp Pro Ala Arg Leu Ile Phe
    370                 375                 380

Asn Thr Gln Thr Gly Pro Ala Ile Val Ala Ser Leu Ile Asp Leu Gly
385                 390                 395                 400

Asp Arg Tyr Arg Leu Leu Val Asn Cys Ile Asp Thr Val Lys Thr Pro
                405                 410                 415

His Ser Leu Pro Lys Leu Pro Val Ala Asn Ala Leu Trp Lys Ala Gln
            420                 425                 430

Pro Asp Leu Pro Thr Ala Ser Glu Ala Trp Ile Leu Ala Gly Gly Ala
        435                 440                 445

His His Thr Val Phe Ser His Ala Leu Asn Leu Asn Asp Met Arg Gln
    450                 455                 460

Phe Ala Glu Met His Asp Ile Glu Ile Thr Val Ile Asp Asn Asp Thr
465                 470                 475                 480

Arg Leu Pro Ala Phe Lys Asp Ala Leu Arg Trp Asn Asp Val Tyr Tyr

485                 490                 495

Gly Phe Arg Arg
            500


<210>    5
<211>    852
<212>    DNA
<213>    Bacillus licheniformis

<400>    5
atgctgacaa atacgaaaaa aatgctgctg gacgcacagg caaacggcta tgcggtgccg      60

gcctttaaca ttcataacct tgaaaccgtg caggccgtga cagaagcggc tgaagcctta     120

aggtcccccg tgattcttgc ggctactcca ggaaccttca gctatgcagg gcgcgactat     180

attcaagcca ttgcagaaac ggccgcggcg cgctgctcga tcccggtcgc cctccatcta     240

gatcatcatg aagaaattga tgatattcgc gagtctttat cattggggac gaaatctgtc     300

atgattgatg cttcgcacca tccatttgcc gagaataccg caattacggc gagtgtcgtt     360

caatatgccc ggcaaaaaga tgccactgta gaagccgagc ttggaaggct cggcggacag     420

gaagacgatt tgatcgtgga tgaagccgac gcttattata ccgatccgga tgcagcaaaa     480

gagttcgtcg agaaaaccgg aatcgattcg ctggcggtgg caatcggtac agcccacggt     540

ctttactcaa aagagcccaa tatcgatttt gagaggctgg ccgcgattca aaaacgggtt     600

tcagtcccgc tcgtcctgca cggcgcctca ggaatttcag agcaagatgt tagaaagtgc     660

atcaagctag gctgtgcaaa agtaaatatc gcgactgaat taaaaattcc tttttcagat     720

aaactgcgcc gatatttgca tgaacacgcc gatgcaagcg atccaagaaa atatatggcc     780

cccgccaaag aagccatgaa aaaaatcgtc caagaaaaga ttttgatgtg catgagcaat     840

gacagatact aa                                                         852


<210>    6
<211>    1503
<212>    DNA
<213>    Escherichia coli

<400>    6
atgacgattt ttgataatta tgaagtgtgg tttgtcattg gcagccagca tctgtatggc      60

ccggaaaccc tgcgtcaggt cacccaacat gccgagcacg tcgttaatgc gctgaatacg     120

gaagcgaaac tgccctgcaa actggtgttg aaaccgctgg gcaccacgcc ggatgaaatc     180

accgctattt gccgcgacgc gaattacgac gatcgttgcg ctggtctggt ggtgtggctg     240

cacaccttct ccccggccaa aatgtggatc aacggcctga ccatgctcaa caaaccgttg     300

ctgcaattcc acacccagtt caacgcggcg ctgccgtggg acagtatcga tatggacttt     360

atgaacctga accagactgc acatggcggt cgcgagttcg gcttcattgg cgcgcgtatg     420

cgtcagcaac atgccgtggt taccggtcac tggcaggata aacaagccca tgagcgtatc 480

ggctcctgga tgcgtcaggc ggtctctaaa caggataccc gtcatctgaa agtctgccga 540

tttggcgata acatgcgtga agtggcggtc accgatggcg ataaagttgc cgcacagatc 600

aagttcggtt tctccgtcaa tacctgggcg gttggcgatc tggtgcaggt ggtgaactcc 660

atcagcgacg gcgatgttaa cgcgctggtc gatgagtacg aaagctgcta caccatgacg 720

cctgccacac aaatccacgg caaaaaacga cagaacgtgc tggaagcggc gcgtattgag 780

ctggggatga agcgtttcct ggaacaaggt ggcttccacg cgttcaccac cacctttgaa 840

gatttgcacg gtctgaaaca gcttcctggt ctggccgtac agcgtctgat gcagcagggt 900

tacggctttg cgggcgaagg cgactggaaa actgccgccc tgcttcgcat catgaaggtg 960

atgtcaaccg gtctgcaggg cggcacctcc tttatggagg actacaccta tcacttcgag 1020

aaaggtaatg acctggtgct cggctcccac atgctggaag tctgcccgtc gatcgccgca 1080

gaagagaaac cgatcctcga cgttcagcat ctcggtattg gtggtaagga cgatcctgcc 1140

cgcctgatct tcaataccca aaccggccca gcgattgtcg ccagcttgat tgatctcggc 1200

gatcgttacc gtctactggt taactgcatc gacacggtga aaacaccgca ctccctgccg 1260

aaactgccgg tggcgaatgc gctgtggaaa gcgcaaccgg atctgccaac tgcttccgaa 1320

gcgtggatcc tcgctggtgg cgcgcaccat accgtcttca gccatgcact gaacctcaac 1380

gatatgcgcc aattcgccga gatgcacgac attgaaatca cggtgattga taacgacaca 1440

cgcctgccag cgtttaaaga cgcgctgcgc tggaacgaag tgtattacgg gtttcgtcgc 1500

taa 1503

<210> 7
<211> 283
<212> PRT
<213> Bacillus licheniformis

<400> 7
Met Leu Thr Asn Thr Lys Lys Met Leu Leu Asp Ala Gln Ala Asn Gly
1               5                   10                  15

Tyr Ala Val Pro Ala Phe Asn Ile His Asn Leu Glu Thr Val Gln Ala
            20                  25                  30

Val Thr Glu Ala Ala Glu Ala Leu Arg Ser Pro Val Ile Leu Ala Ala
        35                  40                  45

Thr Pro Gly Thr Phe Ser Tyr Ala Gly Arg Asp Tyr Ile Gln Ala Ile
    50                  55                  60

Ala Glu Thr Ala Ala Ala Arg Cys Ser Ile Pro Val Ala Leu His Leu
65                  70                  75                  80

Asp His His Glu Glu Ile Asp Asp Ile Arg Glu Ser Leu Ser Leu Gly
                85                  90                  95

Thr Lys Ser Val Met Ile Asp Ala Ser His His Pro Phe Ala Glu Asn
            100                 105                 110

Thr Ala Ile Thr Ala Ser Val Val Gln Tyr Ala Arg Gln Lys Asp Ala
        115                 120                 125

Thr Val Glu Ala Glu Leu Gly Arg Leu Gly Gly Gln Glu Asp Asp Leu
    130                 135                 140

Ile Val Asp Glu Ala Asp Ala Tyr Tyr Thr Asp Pro Asp Ala Ala Lys
145                 150                 155                 160

Glu Phe Val Glu Lys Thr Gly Ile Asp Ser Leu Ala Val Ala Ile Gly
                165                 170                 175

Thr Ala His Gly Leu Tyr Ser Lys Glu Pro Asn Ile Asp Phe Glu Arg
            180                 185                 190

Leu Ala Ala Ile Gln Lys Arg Val Ser Val Pro Leu Val Leu His Gly
        195                 200                 205

Ala Ser Gly Ile Ser Glu Gln Asp Val Arg Lys Cys Ile Lys Leu Gly
    210                 215                 220

Cys Ala Lys Val Asn Ile Ala Thr Glu Leu Lys Ile Pro Phe Ser Asp
225                 230                 235                 240

Lys Leu Arg Arg Tyr Leu His Glu His Ala Asp Ala Ser Asp Pro Arg
                245                 250                 255

Lys Tyr Met Ala Pro Ala Lys Glu Ala Met Lys Lys Ile Val Gln Glu
            260                 265                 270

Lys Ile Leu Met Cys Met Ser Asn Asp Arg Tyr
        275                 280

<210> 8
<211> 500
<212> PRT
<213> Escherichia coli

<400> 8
Met Thr Ile Phe Asp Asn Tyr Glu Val Trp Phe Val Ile Gly Ser Gln
1               5                   10                  15

His Leu Tyr Gly Pro Glu Thr Leu Arg Gln Val Thr Gln His Ala Glu
            20                  25                  30

His Val Val Asn Ala Leu Asn Thr Glu Ala Lys Leu Pro Cys Lys Leu
        35                  40                  45

Val Leu Lys Pro Leu Gly Thr Thr Pro Asp Glu Ile Thr Ala Ile Cys
    50                  55                  60

Arg Asp Ala Asn Tyr Asp Asp Arg Cys Ala Gly Leu Val Val Trp Leu
65                  70                  75                  80

His Thr Phe Ser Pro Ala Lys Met Trp Ile Asn Gly Leu Thr Met Leu
                85                  90                  95

Asn Lys Pro Leu Leu Gln Phe His Thr Gln Phe Asn Ala Ala Leu Pro
            100                 105                 110

Trp Asp Ser Ile Asp Met Asp Phe Met Asn Leu Asn Gln Thr Ala His
        115                 120                 125

Gly Gly Arg Glu Phe Gly Phe Ile Gly Ala Arg Met Arg Gln Gln His
    130                 135                 140

Ala Val Val Thr Gly His Trp Gln Asp Lys Gln Ala His Glu Arg Ile
145                 150                 155                 160

Gly Ser Trp Met Arg Gln Ala Val Ser Lys Gln Asp Thr Arg His Leu
                165                 170                 175

Lys Val Cys Arg Phe Gly Asp Asn Met Arg Glu Val Ala Val Thr Asp
            180                 185                 190

Gly Asp Lys Val Ala Ala Gln Ile Lys Phe Gly Phe Ser Val Asn Thr
        195                 200                 205

Trp Ala Val Gly Asp Leu Val Gln Val Val Asn Ser Ile Ser Asp Gly
    210                 215                 220

Asp Val Asn Ala Leu Val Asp Glu Tyr Glu Ser Cys Tyr Thr Met Thr
225                 230                 235                 240

Pro Ala Thr Gln Ile His Gly Lys Lys Arg Gln Asn Val Leu Glu Ala
                245                 250                 255

Ala Arg Ile Glu Leu Gly Met Lys Arg Phe Leu Glu Gln Gly Gly Phe
            260                 265                 270

His Ala Phe Thr Thr Thr Phe Glu Asp Leu His Gly Leu Lys Gln Leu
        275                 280                 285

Pro Gly Leu Ala Val Gln Arg Leu Met Gln Gln Gly Tyr Gly Phe Ala
    290                 295                 300

Gly Glu Gly Asp Trp Lys Thr Ala Ala Leu Leu Arg Ile Met Lys Val
305                 310                 315                 320

Met Ser Thr Gly Leu Gln Gly Gly Thr Ser Phe Met Glu Asp Tyr Thr
                325                 330                 335

Tyr His Phe Glu Lys Gly Asn Asp Leu Val Leu Gly Ser His Met Leu
            340                 345                 350

Glu Val Cys Pro Ser Ile Ala Ala Glu Glu Lys Pro Ile Leu Asp Val
        355                 360                 365

Gln His Leu Gly Ile Gly Gly Lys Asp Asp Pro Ala Arg Leu Ile Phe
    370                 375                 380

Asn Thr Gln Thr Gly Pro Ala Ile Val Ala Ser Leu Ile Asp Leu Gly
385                 390                 395                 400

Asp Arg Tyr Arg Leu Leu Val Asn Cys Ile Asp Thr Val Lys Thr Pro
                405                 410                 415

His Ser Leu Pro Lys Leu Pro Val Ala Asn Ala Leu Trp Lys Ala Gln
            420                 425                 430

Pro Asp Leu Pro Thr Ala Ser Glu Ala Trp Ile Leu Ala Gly Gly Ala
        435                 440                 445

His His Thr Val Phe Ser His Ala Leu Asn Leu Asn Asp Met Arg Gln
    450                 455                 460

Phe Ala Glu Met His Asp Ile Glu Ile Thr Val Ile Asp Asn Asp Thr
465                 470                 475                 480

Arg Leu Pro Ala Phe Lys Asp Ala Leu Arg Trp Asn Glu Val Tyr Tyr
                485                 490                 495

Gly Phe Arg Arg
            500


<210>    9
<211>    33
<212>    DNA
<213>    Artificial Sequence

<220>
<223>    ECAI NdeI Forward primer


<400>    9
catatgacga tttttgataa ttatgaagtg tgg                                    33


<210>    10
<211>    28
<212>    DNA
<213>    Artificial Sequence

<220>
<223>    ECAI HindIII reverse  primer


<400>    10
aagcttttag cgacgaaacc cgtaatac                                          28


<210>    11
<211>    28
<212>    DNA
<213>    Artificial Sequence

<220>
<223>    Bli03552 forward  primer


<400>    11
catatgctga caaatacgaa aaaaatgc                                          28


<210>    12
<211>    29
<212>    DNA
<213>    Artificial Sequence

<220>
<223>    Bli03552 gatY XhoI Reverse  primer


<400>    12

ctcgagttag tatctgtcat tgctcatgc 29